# EUROPÄISCHE PATENTSCHRIFT

(11) **EP 0 783 522 B1**
(45) Veröffentlichungstag und Bekanntmachung des Hinweises auf die Patenterteilung: **05.12.2001**
(21) Anmeldenummer: 95934629.7
(22) Anmeldetag: 22.09.1995
(51) Int. Cl.: C07K 14/635, C07K 16/24, G01N 33/78

(54) **PEPTIDE AUS DER SEQUENZ DES hPTH (1-37)**
PEPTIDES FROM THE hPTH SEQUENCE (1-37)
PEPTIDES DE LA SEQUENCE DE hPTH (1-37)

(30) Priorität: 28.09.1994 DE 4434551
(43) Veröffentlichungstag der Anmeldung: 16.07.1997
(73) Patentinhaber: Pharis Biotec GmbH, 30625 Hannover (DE)
(72) Erfinder: FORSSMANN, Wolf-Georg, D-30625 (DE); ADERMANN, Knut, D-30177 Hannover (DE); HOCK, Dieter, D-74924 Neckarsbischofsheim (DE); MÄGERLEIN, Markus, D-63785 Obernburg (DE)
(74) Vertreter: Godemeyer, Thomas, Dr.
(86) Internationale Anmeldenummer: EP9503757
(87) Internationale Veröffentlichungsnummer: WO9610041

(56) Entgegenhaltungen:
- WO-A-91/06564
- WO-A-94/03201
- DE-A- 3 347 548
- FR-A- 2 550 204
- CHEMICAL ABSTRACTS, vol. 96, no. 21, 24.Mai 1982 Columbus, Ohio, US; abstract no. 174594, NAKAMURA, RYUICHI ET AL 'Action of fragments of human parathyroid hormone on blood pressure in rats' & ENDOCRINOL. JPN. (1981), 28(4), 547-9 CODEN: ECJPAE;ISSN: 0013-7219, 1981
- CHEMICAL ABSTRACTS, vol. 96, no. 5, 1.Februar 1982 Columbus, Ohio, US; abstract no. 29060, NUSSBAUM, SAMUEL R. ET AL 'Monoclonal antibodies directed against the biologically active region of parathyroid hormone' & MONOCLONAL ANTIBODIES ENDOCR. RES. (1981), 181-92. EDITOR(S): FELLOWS, ROBERT E.;EISENBARTH, GEORGE S. PUBLISHER: RAVEN, NEW YORK, N. Y. CODEN: 46PAAZ, 1981
- JOURNAL OF IMMUNOASSAY, Bd. 13, Nr. 1, 1992 NEW YORK, US, Seiten 1-13, J. TAMPE ET AL. 'Characterization of Antibodies against Human N-Terminal Parathyroid Hormone by Epitope Mapping' in der Anmeldung erwähnt
- J. PHARMACOL. EXP. THER. (1981), 216(3), 567-71 CODEN: JPETAB;ISSN: 0022-3565, 1981 PANG, PETER K. T. ET AL 'Hypotensive action of synthetic fragments of parathyroid hormone'
- CHEMICAL ABSTRACTS, vol. 95, no. 17, 26.Oktober 1981 Columbus, Ohio, US; abstract no. 144316, HASHIMOTO, KEITARO ET AL 'Effects of parathyroid hormone and related polypeptides on the heart and coronary circulation of dogs' & J. CARDIOVASC. PHARMACOL. (1981), 3(4), 668-76 CODEN: JCPCDT;ISSN: 0160-2446, 1981
- E. Wingender et al. 'Structure- Function Relationship in Parathyroid Hormone' in: Advances in Protein Design, International Workshop 1988, GBF Monographs, Vol. 12, ed. by H. Blöcker, J. Collins, R.D. Schmid , D. Schomburg; pub. by VCH, 1988, p. 167-176
- ADVANCES IN EXPERIMENTAL MEDICINE AND BIOLOGY, Bd. 208, 1986 NEW YORK, US, Seiten 315-327, L.H. CAPORALE AND M. ROSENBLATT 'Parathyroid Hormone Antagonists effective in vivo'

## Beschreibung

Die vorliegende Erfindung betrifft Peptide aus der Sequenz des hPTH (1-37), ein Diagnostikum erhältlich durch Immunisierung von Tieren mit den Peptiden, Antikörper oder deren Fragmente erhältlich durch Immunisierung von Tieren mit den Peptiden sowie die Verwendung der Peptide zur Herstellung eines Mittels zur Diagnose von biologisch aktivem h-PTH.

Die Sequenz des hPTH ist bereits bekannt aus Advances in Protein Design, International Workshop 1988, GBF Monographs, Vol. 12, Wingender et al., Seite 170, Fig. 1.

Einige Peptide aus der Sequenz des hPTH sind bereits im Stand der Technik beschrieben (WO-A- 94 03201, FR-A- 2 550 204, Journal of Immunoessay, Bd. 13, J. Tampe et al.; Journal of Pharmacology and Exp. Ther. (1981), 216 (3) Pang et al.; Chemical Abstracts, Vol. 96, No. 21, R. Nakamura et al.).

Humanes Parathormon (hPTH), ein lineares Polypeptid aus 84 Aminosäuren, spielt eine wichtige Rolle in der Regulation des Calciumstoffwechsels. Der Metabolismus dieses Hormons führt zu einer großen Zahl C-terminaler Fragmente, deren biologische Funktion noch nicht geklärt ist. Als zirkulierendes N-terminales Fragment ist das hPTH 1-37 festgelegt (EP-A 0 349 545). Dieses Fragment besitzt die volle biologische Aktivität des Gesamthormons. Diese nimmt allerdings bei Verlust der ersten Aminosäure, Serin, deutlich ab und geht ohne die ersten beiden Aminosäuren, Serin und Valin, völlig verloren.

Für das intakte Hormon hPTH 1-84 und für das N-terminale Fragment werden Serumkonzentrationen im Bereich von 10⁻¹² mol/l gemessen. Zur Bestimmung solch niedriger Konzentrationen bedient man sich immunologischer Meßverfahren. Die validesten Ergebnisse liefern hierbei Meßverfahren nach dem Doppelantikörper oder Sandwich Prinzip (z.B. Two-site Radioimmunometric Assay, IRMA oder Sandwich Enzym Linked Immuno Sorbent Assay, Sandwich ELISA). Solche Assays sind für hPTH 1-84 kommerziell erhältlich. Zur Messung von hPTH 1-34 ist ein Assay nach dem Doppelantikörper-Prinzip nicht bekannt.

Hierfür sind zwei Antikörper notwendig. Diese müssen, um eine gegenseitige sterische Hinderung zu vermeiden, Epitope des Antigens erkennen, die in ausreichender Entfernung zueinander liegen. Bei Immunisierung mit dem intakten Antigen erhält man ein heterogenes Gemisch unterschiedlicher Antikörper, die für einen Sandwich-Assay erst aufwendig gereinigt werden müssen. Zwar war es bisher möglich aufgrund theoretischer Berechnungen nach B.A. Jameson & H. Wolf, The antigenic index: a novel algorithm for predicting antigenic determinants, CABIOS 4, p 181-186, 1988 am N-Terminus eine bevorzugte immunogen wirkende Sequenz im Bereich der Aminosäuren 7 - 14 festzustellen. Eine Immunisierung mit N-terminalen Fragmenten nach etablierten Methoden führt in erster Linie zu Antikörpern, die, wie für hPTH 1-34 beschrieben (J. Tampe, P. Brozio, H.E. Manneck, A. Mißbichler, E. Blind, K.B. Müller, H. Schmidt-Gayk und F.P. Armbruster; Characterisation of antibodies against human N-terminal parathyroid hormon by epitope mapping; J. Immunoassay 13 S. 1-13, 1992), in dem Bereich dieser Aminosäuren binden. Diese Antikörper können aber nicht zwischen biologisch aktiven und biologisch inaktiven PTH 1-84 oder Fragmenten davon, denen die ersten beiden Aminosäuren Serin und Valin fehlen, unterscheiden.

Das der Erfindung zugrunde liegende technische Problem besteht darin, Peptide anzugeben, mit deren Hilfe die oben genannten Nachteile der Diagnose von biologisch aktivem h-PTH beseitigt werden können.

Das angesprochene technische Problem wird überraschenderweise gelöst durch Verwendung der Peptide aus der Sequenz des humanen Parathyroidhormons (hPTH) mit der Sequenz 
hPTH 1-10
NH2-Ser1-Val2-Ser3-Glu4-Ile5-Gln6-Leu7-Met8-His9-Asn10-OH SEQ ID NO 1
 
hPTH 1-9
NH2-Ser1-Val2-Ser3-Glu4-Ile5-Gln6-Leu7-Met8-His9-OH SEQ ID No 2
 
hPTH 1-8
NH2-Ser1-Val2-Ser3-Glu4-Ile5-Gln6-Leu7-Met8-OH SEQ ID NO 3
 
hPTH 1-7
NH2-Ser1-Val2-Ser3-Glu4-Ile5-Gln6-Leu7-OH SEQ ID NO 4
 
hPTH 1-6
NH2-Ser1-Val2-Ser3-Glu4-Ile5-Gln6-OH SEQ ID NO 5
 
hPTH 1-5
NH2-Ser1-Val2-Ser3-Glu4-Ile5-OH SEQ ID NO 6
 zur Herstellung von Antikörpern oder Antikörperfragmenten zur Diagnose von biologisch aktivem hPTH (1-37).

In einer bevorzugten Verwendung können die Peptide am N-terminalen Ende, in der Seitenkette und/oder am C-terminalen Ende modifiziert sein, und zwar in Form von Acetylierungs-, Amidierungs-, Phosphorylierungs- und/oder Glycosylierungsprodukten und/oder an Carrierproteine ausgewählt aus der Gruppe Hämocyanin, Thyroglobulin, Rinderserumalbumin, Ovalbumin oder Mausserumalbumin gebunden sein. Die Bindung an die Carrierproteine erfolgt vorzugsweise durch Carbodiimid oder Formaldehyd.

Das technische Problem wird weiterhin gelöst durch einen Antikörper oder ein Antikörperfragment, die ein Peptid spezifisch erkennen und binden, wobei das Peptid ausgewählt ist aus der Gruppe SEQ ID NO 1 bis 4 und 6.

Das technische Problem wird auch gelöst, durch ein Diagnostikum zum Nachweis von biologisch aktivem humanen Parathyroidhormon (hPTH (1 - 37)) umfassend einen Antikörper oder ein Antikörperfragment, die ein Peptid spezifisch erkennen und binden, wobei das Peptid ausgewählt ist aus der Gruppe SEQ ID NO 1 bis 4 und 6 sowie durch die Bereitstellung eines Diagnostikums gemäß Ansprüchen 6 und 7.

Zudem wird das technische Problem gelöst durch ein in vitro Verfahren zum Nachweis von biologisch aktivem Parathyroidhormon (hPTH (1 - 37)) unter Verwendung des Antikörpers gemäß Anspruch 3 oder 4, bzw. einem in vitro Verfahren, in dem zusätzlich zu den Antikörpern gemäß Ansprüchen 3 oder 4 ein zweiter Antikörper oder Antikörperfragment verwendet wird, der im Bereich der Aminosäurepositionen 9-15 oder 30-37 des humanen Parathormons bindet, wobei beide Antikörper Epitope des Antigens erkennen, die in ausreichender Entfernüng zueinander liegen, um eine gegenseitige sterische Hinderung zu vermeiden.

Die genannten Sequenzen repräsentieren in ihrer Primärstruktur wesentliche Merkmale der Sekundärstruktur, wie sich durch NMR-Daten unterstützend belegen läßt. Voraussetzung dazu war ein Festlegung der Sekundärstruktur für PTH 1-37 in physiologischer Lösung.

Die genannten strukturell auffälligen Bereiche wirken gut immunogen. Es werden Antikörper gebildet, die an den ersten Aminosäuren des N-Terminus binden. Bereits das Fehlen von zwei Aminosäuren führt zu einem erheblichen Affinitätsverlust. Da diese Aminosäuren zur Ausübung der biologischen Wirkung unerläßlich sind, ist es mit den erfindungsgemäßen Peptiden möglich Antikörper zu erhalten, die nur hPTH und Fragmente davon erkennen, die biologisch aktiv sind.

Weiterhin sind Antikörper herstellbar, die den midregionalen Bereich 9-15 detektieren, und Antikörper, die C-terminal im Bereich der Aminosäuren 30-37 binden. Erfindungsgemäß können also Antikörper gegen Bereiche des hPTH 1-37 produziert werden, die aufgrund theoretischer Berechnungen im Gesamtmolekül nicht immunogen wirken. Diese Bereiche liegen zudem soweit auseinander, daß keine sterische Hinderung vorliegt, die ein gleichzeitiges Binden zweier Antikörper verhindern würde.

Die Peptide können verwendet werden, um ein Diagnostikum herzustellen. Das er findungsgemäße Diagnostikum ist dabei erhältlich durch an sich bekannte Immunisierung von Tieren mit mindestens einem der Peptide. Nach der Immunisierung kann aus den immunisierten Tieren eine Immunoglobulin-Fraktion isoliert werden, die Antikörper-Fraktionen enthält, welche einen Antikörper-Titer gegen mindestens eines der Peptide aufweisen. Die so erhaltenen Antikörpern sind ebenfalls Gegenstand der vorliegenden Erfindung. In einer alternativen Ausführungsform können neben den vollständigen Antikörpern bestehend aus F_{ab} und F_{c} auch deren Fragmente wie F_{ab} oder Fragmente der Antikörper verwendet werden, welche die Idiotypen zu den Epitopen der Peptide sind.

Die Peptide sind zur Herstellung eines Mittels zur Diagnose von biologisch aktiven h-PTH (1-37) geeignet.

Die Erfindung wird anhand der folgenden Beispiele näher beschrieben:

### Beispiel 1

### Festphasensynthese der Peptide:

Das Verfahren zur Synthese der Peptide beruht auf der Peptidsynthese am festen Träger. Die C-terminale Aminosäure wird jeweils in Gegenwart von Dicyclohexylcarbodiimid und Dimethylaminopyridin an das Trägermaterial gebunden. Als Trägermaterial für die Synthesen werden Wang-Harz oder entsprechende Harze eingesetzt.

Folgende L-Aminosäure-Derivate werden für die Synthese der Sequenz, ausgehend vom aufgeführten Peptidyl-Harz, verwendet: a) hPTH 1-10: Fmoc-Asn(Trt)-Wang-Harz, Fmoc-His(Trt)-OH, Fmoc-Met-OH, Fmoc-Leu-OH, Fmoc-Gln(Trt)-OH, Fmoc-Ile-OH, Fmoc-Glu(OtBu)-OH, Fmoc-Ser(tBu)-OH, Fmoc-Val-OH, Boc-Ser(tBu)-OH. b) hPTH 9-18: Fmoc-Met-Wang-Harz, Fmoc-Ser(tBu)-OH, Fmoc-Asn(Trt)-OH, Fmoc-Leu-OH, Fmoc-His(Trt)-OH, Fmoc-Lys(Boc)-OH, Fmoc-Gly-OH, Fmoc-Leu-OH, Fmoc-Asn(Trt)-OH, Boc-His(Trt)-OH. c) hPTH 24-37: Fmoc-Leu-Wang-Harz, Fmoc-Ala-OH, Fmoc-Val-OH, Fmoc-Phe-OH, Fmoc-Asn(Trt)-OH, Fmoc-His(Trt)-OH, Fmoc-Val-OH, Fmoc-Asp(OtBu)-OH, Fmoc-Gln(Trt)-OH, Fmoc-Leu-OH, Fmoc-Lys(Boc)-OH, Fmoc-Lys(Boc)-OH, Fmoc-Arg(Pmc)-OH, Fmoc-Leu-OH.

Die Synthese kann durch in situ-Aktivierung mit 2-(1H-Benzotriazol-1-yl)-1,1,3,3,-tetramethyluroniumtetrafluoroborat (TBTU) oder dessen Derivaten oder mit Benzotriazol-1-yl-oxy-tris-(dimethylamino)-phosphoniumhexafluorophosphat (BOP) oder dessen Derivaten in Gegenwart von Diisopropylethylamin oder N-Methylmorpholin und 1-Hydroxybenzotriazol durchgeführt werden, wobei während der Kupplungen in N,N-Dimethylformamid, N,N-Dimethylacetamid oder N-Methylpyrrolidon ein vier- bis zehnfacher Überschuß der Fmoc-L-Aminosäure verwendet wird. Die Abspaltungen der Fmoc-Gruppen werden mit 20% Piperidin oder 2% Piperidin und 2% 1,8-Diazbicyclo[5,4,0]undec-7-en (DBU) in N,N-Dimethylformamid, N,N-Dimethylacetamid oder N-Methylpyrrolidon durchgeführt. Nach der Synthese werden die Harze mit 2-Propanol und Dichlormethan gewaschen und im Hochvakuum bis zur Gewichtskonstanz getrocknet.

Zur Abspaltung vom Träger und Entblockierung wird das Peptidyl-Harz 30-90 Minuten bei Raumtemperatur mit Trifluoressigsäure, die 5% Scavenger, Wasser, Ethandiol, Phenol oder Thioanisol, enthält, umgesetzt, filtriert, mit Trifluoressigsäure gewaschen und anschließend mit tert-Butylmethylether ausgefällt. Der Niederschlag wird aus wäßriger Lösung lyophilisiert.

### Beispiel 2

### Reinigung und Analyse

Die Reinigung der Rohprodukte erfolgt chromatographisch über eine C18-Reverse-Phase-Säule (10µm, Puffer A: 0,01 N HCl in Wasser; Puffer B: 20% Isopropanol, 30 % Methanol, 50% Wasser, 0,01 N HCl; Gradient: 10-80% in 60 Minuten; Detektion 230 nm).

Die Reinheit der Produkte wird durch Massenspektrometrie und C18-Reverse-Phase-Chromatographie bestimmt.

### Beispiel 3

### Kopplung an Carrierprotein

Als Carrierprotein werden Hämocyanin, Thyroglobulin, Rinderserumalbumin, Ovalbumin oder Mausserumalbumin verwendet. Die Kopplung erfolgt nach der Carbodiimid-Methode über Carboxylgruppen des Peptides. Das Peptid wird in wässriger Lösung durch 5 minütige Umsetzung mit 1-Ethyl-3-(3-dimethylaminopropyl) carbodiimid-Hydrochlorid aktiviert. Die Kopplung erfolgt durch Zugabe des aktivierten Peptides zu einer wässrigen Lösung des Carriers. Das molare Verhälnis beträgt 1 Peptid auf 50 Aminosäuren des Carrierproteins. Die Umsetzung dauert 4 Stunden.
Die Reaktion wird durch Zugabe von Natriumacetat in einer Endkonzentration von 100 mM gestoppt. Man läßt eine Stunde inkubieren.

Die Abtrennung des Protein-Peptid Konjugates vom Peptid erfolgt durch wiederholte Dialyse gegen 100 mM Phosphatpuffer pH 7,2.

### Beispiel 4

### Synthese der Multiple Antigenic Peptides (MAP)

Die dreifache Lysin-Verzweigung wird erreicht, indem an C-terminales Alanin , gebunden an Wang-Harz, in drei Kupplungszyklen jeweils Fmoc-L-Lysin(Fmoc)-OH gebunden wird. Durch Abspaltung mit Piperidin werden danach acht freie Aminofunktionen erhalten, an denen die Sequenzen des humanen Parathormons nach obiger Beschreibung synthetisiert werden.

### Beispiel 5

### Immunisierung

Für die Erstimmunisierung werden pro kg Körpergewicht des zu immunisierenden Tieres 125 µg des Carrier-Peptid Konjugates bzw. MAP in 250 ml Wasser gelöst und mit 250 µl kompletten Freund'schen Adjuvans emulgiert. Die Emulsion wird über den Rücken verteilt in 10 Portionen s.c. appliziert.

Das Boostern erfolgt nach 2-4 Wochen analog. Hierbei wird lediglich das komplette Freund'sche Adjuvans durch inkomplettes Freund'sches Adjuvans ersetzt.

## Patentansprüche

1. Verwendung der Peptide aus der Sequenz des humanen Parathyroidhormons (hPTH) mit der Sequenz 
hPTH 1-10
NH2-Ser1-Val2-Ser3-Glu4-Ile5-Gln6-Leu7-Met8-His9-Asn10-OH SEQ ID NO 1
 
hPTH 1-9
NH2-Ser1-Val2-Ser3-Glu4-Ile5-Gln6-Leu7-Met8-His9-OH SEQ ID No 2
 
hPTH 1-8
NH2-Ser1-Val2-Ser3-Glu4-Ile5-Gln6-Leu7-Met8-OH SEQ ID NO 3
 
hPTH 1-7
NH2-Ser1-Val2-Ser3-Glu4-Ile5-Gln6-Leu7-OH SEQ ID NO 4
 
hPTH 1-6
NH2-Ser1-Val2-Ser3-Glu4-Ile5-Gln6-OH SEQ ID NO 5
 
hPTH 1-5
NH2-Ser1-Val2-Ser3-Glu4-Ile5-OH SEQ ID NO 6
 zur Herstellung von Antikörpern oder Antikörperfragmenten zur Diagnose von biologisch aktivem hPTH (1-37).

2. Verwendung nach Anspruch 1, **dadurch gekennzeichnet, daß** die Peptide, am N-terminalen Ende, in der Seitenkette und/oder am C-terminalen Ende modifiziert sind in Form von Acetylierungs-, Amidierungs-, Phosphorylierungs- und/oder Glycosylierungsprodukten, und/oder gebunden sind an Carrierproteine ausgewählt aus der Gruppe Hämocyanin, Thyroglobulin, Rinderserumalbumin, Ovalalbumin oder Mausserumalbumin.

3. Antikörper oder Antikörperfragment, die ein Peptid spezifisch erkennen und binden, wobei das Peptid ausgewählt ist aus der Gruppe SEQ ID NO 1 bis 4 und 6.

4. Antikörper nach Anspruch 3, **dadurch gekennzeichnet, daß** der Antikörper oder das Antikörperfragment Peptide erkennen, die am N-terminalen Ende, in der Seitenkette und/oder am C-terminalen Ende modifiziert sind in Form von Acetylierungs-, Amidierungs-, Phosphorylierungs- und/oder Glycosylierungsprodukten, und/oder gebunden sind an Carrierproteine ausgewählt aus der Gruppe Hämocyanin, Thyroglobulin, Rinderserumalbumin, Ovalalbumin oder Mausserumalbumin.

5. Diagnostikum zum Nachweis von biologisch aktivem humanen Parathyroidhormon (hPTH (1-37)) umfassend einen Antikörper oder ein Antikörperfragment, die ein Peptid spezifisch erkennen und binden, wobei das Peptid ausgewählt ist aus der Gruppe SEQ ID NO 1 bis 4 und 6.

6. Diagnostikum nach Anspruch 5, **dadurch gekennzeichnet, daß** zusätzlich ein zweiter Antikörper oder Antikörperfragment enthalten ist, der im Bereich der Aminosäurepositionen 9-15 oder 30-37 des humanen Parathyroidhormons bindet, wobei beide Antikörper Epitope des Antigens erkennen, die in ausreichender Entfernung zueinander liegen, um eine gegenseitige sterische Hinderung zu vermeiden.

7. Diagnostikum nach Anspruch 5 oder 6, **dadurch gekennzeichnet, daß** der Antikörper oder das Antikörperfragment Peptide erkennen, die am N-terminalen Ende, in der Seitenkette und/oder am C-terminalen Ende modifiziert sind in Form von Acetylierungs-, Amidierungs-, Phosphorylierungs- und/oder Glycosylierungsprodukten, und/oder gebunden sind an Carrierproteine ausgewählt aus der Gruppe Hämocyanin, Thyroglobulin, Rinderserumalbumin, Ovalalbumin oder Mausserumalbumin.

8. Verfahren zum Nachweis von biologisch aktivem humanen Parathyroidhormon (hPTH (1-37)) in vitro unter Verwendung des Antikörpers gemäß Anspruch 3 oder 4.

9. Verfahren nach Anspruch 8, **dadurch gekennzeichnet, daß** zusätzlich ein zweiter Antikörper oder Antikörperfragment verwendet wird, der im Bereich der Aminosäurepositionen 9-15 oder 30-37 des humanen Parathyroidhormons bindet, wobei beide Antikörper Epitope des Antigens erkennen, die in ausreichender Entfernung zueinander liegen, um eine gegenseitige sterische Hinderung zu vermeiden.

10. Verfahren nach Anspruch 8 oder 9, **dadurch gekennzeichnet, daß** der Antikörper oder das Antikörperfragment Peptide erkennen, die am N-terminalen Ende, in der Seitenkette und/oder am C-terminalen Ende modifiziert sind in Form von Acetylierungs-, Amidierungs-, Phosphorylierungs- und/oder Glycosylierungsprodukten, und/oder gebunden sind an Carrierproteine ausgewählt aus der Gruppe Hämocyanin, Thyroglobulin, Rinderserumalbumin, Ovalalbumin oder Mausserumalbumin.

## Claims

1. Use of peptides from the sequence of human parathyroid hormone (hPTH), having the sequence 
hPTH 1-10
NH2-Ser1-Val2-Ser3-Glu4-Ile5-Gln6-Leu7-Met8-His9-Asn10-OH SEQ ID NO 1
 
hPTH 1-9
NH2-Ser1-Val2-Ser3-Glu4-Ile5-Gln6-Leu7-Met8-His9-OH SEQ ID NO 2
 
hPTH 1-8
NH2-Ser1-Val2-Ser3-Glu4-Ile5-Gln6-Leu7-Met8-OH SEQ ID NO 3
 
hPTH 1-7
NH2-Ser1-Val2-Ser3-Glu4-Ile5-Gln6-Leu7-OH SEQ ID NO4
 
hPTH 1-6
NH2-Ser1-Val2-Ser3-Glu4-Ile5-Gln6-OH SEQ ID NO5
 
hPTH 1-5
NH2-Ser1-Val2-Ser3-Glu4-Ile5-OH SEQ ID NO6
 in the production of antibodies or antibody fragments for diagnosing biologically active hPTH (1-37).

2. The use according to claim 1, **characterized in that** the peptides have been modified at the N-terminal end, in the side chain and/or the C-terminal end thereof in the form of acetylation, amidation, phosphorylation and/or glycosylation products and/or bound to carrier proteins selected from the group of hemocyanin, thyroglobulin, bovine serum albumin, ovalbumin, or mouse serum albumin.

3. An antibody or antibody fragment specifically identifying and binding a peptide, said peptide being selected from the group of SEQ ID NO 1 through 4 and 6.

4. The antibody according to claim 3, **characterized in that** the antibody or antibody fragment will identify peptides modified at the N-terminal end, in the side chain and/or the C-terminal end thereof in the form of acetylation, amidation, phosphorylation and/or glycosylation products and/or bound to carrier proteins selected from the group of hemocyanin, thyroglobulin, bovine serum albumin, ovalbumin, or mouse serum albumin.

5. A diagnostic agent for detecting biologically active human parathyroid hormone (hPTH (1-37)), said agent comprising an antibody or antibody fragment specifically identifying and binding a peptide, said peptide being selected from the group of SEQ ID NO 1 through 4 and 6.

6. The diagnostic agent according to claim 5, **characterized in that** a second antibody or antibody fragment is included in addition, which binds in the region of amino acid positions 9-15 or 30-37 of human parathyroid hormone, both antibodies being capable of identifying epitopes of the antigen located at a sufficient distance from each other so as to avoid mutual steric hindrance.

7. The diagnostic agent according to claim 5 or 6, **characterized in that** the antibody or antibody fragment will identify peptides modified at the N-terminal end, in the side chain and/or the C-terminal end thereof in the form of acetylation, amidation, phosphorylation and/or glycosylation products and/or bound to carrier proteins selected from the group of hemocyanin, thyroglobulin, bovine serum albumin, ovalbumin, or mouse serum albumin.

8. A method of detecting biologically active human parathyroid hormone (hPTH (1-37)) *in vitro,* using the antibody according to claim 3 or 4.

9. The method as claimed in claim 8, **characterized in that** a second antibody or antibody fragment is used in addition, which binds in the region of amino acid positions 9-15 or 30-37 of human parathyroid hormone, both antibodies being capable of identifying epitopes of the antigen located at a sufficient distance from each other so as to avoid mutual steric hindrance.

10. The method according to claim 8 or 9, **characterized in that** the antibody or antibody fragment will identify peptides modified at the N-terminal end, in the side chain and/or the C-terminal end thereof in the form of acetylation, amidation, phosphorylation and/or glycosylation products and/or bound to carrier proteins selected from the group of hemocyanin, thyroglobulin, bovine serum albumin, ovalbumin, or mouse serum albumin.

## Revendications

1. Utilisation de peptides contenus dans la séquence de l'hormone parathyroïdienne (hPTH) humaine, qui ont pour séquence 
hPTH 1-10
NH2-Ser1-Val2-Ser3-Glu4-Ile5-Gln6-Leu7-Met8-His9-Asn10-OH SEQ ID N° 1
 
hPTH 1-9
NH2-Ser1-Val2-Ser3-Glu4-Ile5-Gln6-Leu7-Met8-His9-OH SEQ ID N° 2
 
hPTH 1-8
NH2-Ser1-Val2-Ser3-Glu4-Ile5-Gln6-Leu7-Met8-OH SEQ ID N° 3
 
hPTH 1-7
NH2-Ser1-Val2-Ser3-Glu4-Ile5-Gln6-Leu7-OH SEQ ID N°4
 
hPTH 1-6
NH2-Ser1-Val2-Ser3-Glu4-Ile5-Gln6-OH SEQ ID N°5
 
hPTH 1-5
NH2-Ser1-Val2-Ser3-Glu4-Ile5-OH SEQ ID N°6
 pour la fabrication d'anticorps ou de fragments d'anticorps pour le diagnostic de l'hPTH (1-37) biologiquement active.

2. Utilisation d'après la revendication 1, **caractérisée en ce que** les peptides, à leur extrémité terminale N, sur leur chaîne latérale, et/ou à leur extrémité terminale C sont modifiés sous forme de produits d'acétylation, d'amidification, de phosphorylation et/ou de glycosylation et/ou sont liés à une protéine porteuse choisie dans le groupe comprenant l'hémocyanine, la thyroglobuline, la sérum albumine de boeuf, l'ovalbumine ou la sérum albumine de souris.

3. Un anticorps ou un fragment d'anticorps, qui reconnaissent et se lient spécifiquement avec un peptide, ledit peptide étant sélectionné dans le groupe des SEQ ID N° 1 à 4 et du SEQ ID N° 6.

4. Anticorps d'après la revendication 3, **caractérisé en ce que** l'anticorps ou le fragment d'anticorps reconnaissent les peptides qui sont modifiés à leur extrémité terminale N, dans leur chaîne latérale, et/ou à leur extrémité terminale C sous forme de produits d'acétylation, d'amidification, de phosphorylation et/ou de glycosylation et/ou qui sont liés à une protéine porteuse choisie dans le groupe comprenant l'hémocyanine, la thyroglobuline, la sérum albumine de boeuf, l'ovalbumine ou la sérum albumine de souris.

5. Diagnostic pour la détection de l'hormone parathyroïdenne humaine biologiquement active (hTPH (1-37)) comprenant un anticorps ou un fragment d'anticorps, qui reconnaissent spécifiquement un peptide et forment une liaison avec celui-ci, le peptide étant sélectionné dans le groupe comportant les N° de SEQ ID de 1 à 4 et le N°6.

6. Diagnostic d'après la revendication 5, **caractérisé en ce qu'**il comporte en plus un deuxième anticorps ou fragment d'anticorps, qui forme une liaison dans une région des acides aminés ayant les positions de 9 à 15, ou de 30 à 37 de l'hormone parathyroïdienne humaine, dans lequel les deux anticorps reconnaissent des épitopes de l'antigène qui sont disposés à un éloignement suffisant l'un de l'autre, pour éviter une gêne stérique mutuelle.

7. Diagnostic d'après la revendication 5 ou 6, **caractérisé en ce que** l'anticorps ou le fragment d'anticorps reconnaissent les peptides, qui sont modifiés à leur extrémité terminale N, à leur chaîne latérale et/ou à leur extrémité latérale C sous forme de produits d'acétylation, d'amidification, de phosphorylation et/ou de glycosylation, et/ou qui sont liés à une protéine porteuse choisie dans le groupe comprenant l'hémocyanine, la thyroglobuline, la sérum albumine de boeuf, l'ovalbumine, ou la sérum albumine de souris.

8. Procédé de détection de l'hormone parathyroïdienne humaine biologiquement active (hTPH (1-37)) *in vitro,* effectué en utilisant l'anticorps d'après la revendication 3 ou 4.

9. Procédé d'après la revendication 8, **caractérisé en ce qu'**en plus, on utilise un deuxième anticorps ou fragment d'anticorps, qui forme une liaison dans une région des acides aminés ayant les positions de 9 à 15, ou de 30 à 37 de l'hormone parathyroïdienne humaine, dans lequel les deux anticorps reconnaissent des épitopes de l'antigène qui sont disposés à un éloignement suffisant l'un de l'autre, pour éviter une gêne stérique mutuelle.

10. Procédé d'après la revendication 8 ou 9, **caractérisé en ce que** l'anticorps ou le fragment d'anticorps reconnaissent les peptides, qui sont modifiés à leur extrémité terminale N, à leur chaîne latérale et/ou à leur extrémité latérale C sous forme de produits d'acétylation, d'amidification, de phosphorylation et/ou de glycosylation, et/ou qui sont liés à une protéine porteuse choisie dans le groupe comprenant l'hémocyanine, la thyroglobuline, la sérum albumine de boeuf, l'ovalbumine, ou la sérum albumine de souris.
